# EUROPEAN PATENT APPLICATION

(11) **EP 2 327 406 A1**
(43) Date of publication of application: **01.06.2011**
(21) Application number: 09806757.2
(22) Date of filing: 14.08.2009
(51) Int. Cl.: A61K 31/40, A61K 47/02, A61K 47/10, A61K 47/12

(54) **STABILIZED PHARMACEUTICAL COMPOSITION**

(30) Priority: 14.08.2008 JP 2008208799
(71) Applicant: Kyorin Pharmaceutical Co., Ltd., Tokyo 101-8311 (JP)
(72) Inventor: UCHIDA Hiroshi, Shimotsuga-gun Tochigi 329-0114 (JP); FUKUDA Mamoru, Shimotsuga-gun Tochigi 329-0114 (JP); ARITOMI Seigo, Shimotsuga-gun Tochigi 329-0114 (JP)
(74) Representative: Vossius & Partner
(86) International application number: PCT/JP2009/064342
(87) International publication number: WO 2010/018866

(57) **Abstract**

A pharmaceutical composition is provided which can easily be prepared and which can prevent decomposition of an aminoacetylpyrrolidine carbonitrile derivative, as an effective component, which derivative is in general instable in a pharmaceutical composition.

More specifically, the pharmaceutical composition comprises an aminoacetyl pyrrolidine carbonitrile derivative represented by the following general formula (1): 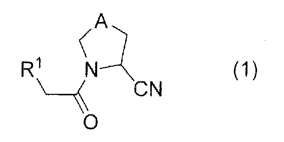 (in the formula (1), A represents CH₂, CHF or CF₂; and R¹ represents a secondary amino group which may have a substituent), and the following components blended with the derivative: (1) a sugar alcohols (2) calcium carbonate, sodium citrate or sodium sulfate; and (3) a neutral low melting point oily and/or fatty substance.

## Description

### Technical Field

The present invention relates to a pharmaceutical composition containing a stabilized aminoacetylpyrrolidine carbonitrile derivative.

### Technical Background

In the production of a pharmaceutical composition, there has often been observed such a phenomenon that the decomposition of the compound included therein is accelerated. For this reason, there have been known a variety of methods for improving the stability of such a compound, as an effective component of a pharmaceutical composition, for instance, a method which comprises adding, to a pharmaceutical composition, an additive such as sodium carbonate as a stabilizing agent (Patent Documents 1 to 8); a method in which a pharmaceutical composition is formed into granules under dry condition (Patent Document 9); a method for applying a coating on a pharmaceutical composition (Patent Document 10); a method in which the principal components of a pharmaceutical composition is dispersed in an oily matrix (Patent Documents 11 to 13); a method which comprises the step of bending a drug through the use of a fused or molten adjuvant having a low melting point (Patent Document 14); and a method which comprises the step of adding a mixture of stearic acid and a drug to preliminarily granulated excipient (Patent Document 15).

However, all of these prior articles do not disclose any means for the stabilization of aminoacetylpyrrolidine carbonitrile derivative and more specifically, they do not disclose that the stability of the compound against decomposition can considerably be improved by the simultaneous use of (1) a sugar alcohol, (2) calcium carbonate, sodium citrate or sodium sulfate, and (3) a neutral low melting point oily and/or fatty substance such as stearyl alcohol, which serve as a stabilizer for the compound.

### Prior Art Literature

### Patent Document

Patent Document 1: WO 2004/032909 Pamphlet;
Patent Document 2: WO 99/20276 Pamphlet;
Patent Document 3: WO 99/20277 Pamphlet;
Patent Document 4: JP-A-11-92369;
Patent Document 5: JP-A-7-285867;
Patent Document 6: JP-A-5-221863;
Patent Document 7: JP-A-6-340530;
Patent Document 8: JP-A-2000-355540;
Patent Document 9: JP-A-2003-128543;
Patent Document 10: JP-A-2006-022039;
Patent Document 11: JP-A-10-324644;
Patent Document 12: WO 00/78318 Pamphlet;
Patent Document 13: JP-A-01-308231;
Patent Document 14: JP-A-05-192094;
Patent Document 15: JP-A.-62-252723.

### Summary of the Invention

### Subject to be attained by the Invention

The present invention relates to a pharmaceutical composition comprising an aminoacetylpyrrolidine carbonitrile derivative as an effective component and in particular, to a pharmaceutical composition which can highly inhibit any decomposition of an aminoacetylpyrrolidine carbonitrile derivative as an effective component and which can easily be prepared.

### Means for attaining the subject:

The inventors of this invention have intensively conducted various studies for the purpose of preparing a pharmaceutical preparation which comprises a compound represented by the following general formula (1) and which can stabilize the compound as an effective component: 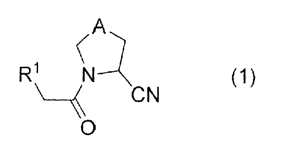 (in the formula (1), A represents CH₂, CHF or CF₂; and R¹ represents a secondary amino group which may have a substituent), have found that the stability of the aminoacetylpyrrolidine carbonitrile derivative can considerably be improved by the simultaneous use of a sugar alcohol; calcium carbonate, sodium citrate or sodium sulfate; and a neutral low melting point oily and/or fatty substance such as stearyl alcohol, as a combination of stabilizers and have thus completed the present invention.
In particular, the inventors have found that the use of a sugar alcohol having a high critical relative humidity is preferred for achieving a satisfactory effect of stabilizing the aminoacetylpyrrolidine carbonitrile derivative. Furthermore, the inventors have likewise found that a further strongly stabilized pharmaceutical preparation containing the compound can be obtained by the addition of calcium carbonate, sodium citrate or sodium sulfate together with a neutral low melting point oily and/or fatty substance, and have thus completed the present invention.
More specifically, the present invention relates to inventions described below in detail:
[1] A pharmaceutical composition comprising, in the stabilized state, an aminoacetylpyrrolidine carbonitrile derivative represented by the following general formula (1): 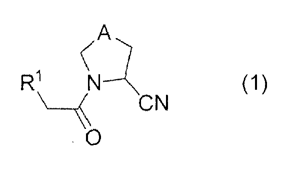 (in the formula (1), A represents CH₂, CHF or CF₂; and R¹ represents a secondary amino group which may have a substituent), wherein it comprises:
   (1) the compound of Formula (1);
   (2) a sugar alcohol;
   (3) one or not less than 2 compounds selected from the group consisting of calcium carbonate, sodium citrate and sodium sulfate; and
   (4) a neutral low melting point oily and/or fatty substance.
[2] The composition as set forth in the foregoing item [1], wherein the sugar alcohol has a critical relative humidity of not less than 70%.
[3] The composition as set forth in the foregoing item [1], wherein the sugar alcohol has a critical relative humidity of not less than 85%.
[4] The composition as set forth in the foregoing item [1], wherein the sugar alcohol is at least one member selected from the group consisting of mannitol, xylitol, erythritol, maltitol, lactitol and sorbitol.
[5] The composition as set forth in the foregoing item [1], wherein the sugar alcohol is mannitol or erythritol.
[6] The composition as set forth in any one of the foregoing items [1] to [5], wherein the neutral low melting point oily and/or fatty substance is a hydrocarbon, a higher alcohol, a fatty acid ester of a polyhydric alcohol, a higher alcohol ether of a polyhydric alcohol or a polymer of an alkylene oxide.
[7] The composition as set forth in the foregoing item [6], wherein the neutral low melting point oily and/or fatty substance is a higher alcohol.
[8] The composition as set forth in the foregoing item [7], wherein the higher alcohol is stearyl alcohol or cetyl alcohol.
[9] The composition as set forth in any one of the foregoing items [1] to [8], wherein the substituent R¹ of the compound of Formula (1) is a secondary amino group represented by the following general formula (2):

   R²-NH- (2)

   (wherein R² represents a C₁ to C₆ alkyl group which may have a substituent, a C₃ to C₁₀ cyclic alkyl group which may have a substituent or a C₂ to C₁₀ cyclic amino group which may have a substituent).
[10] The composition as set forth in any one of the foregoing items [1] to [9], wherein the compound of Formula (1) is an aminoacetylpyrrolidine carbonitrile derivative represented by the following general formula (3): 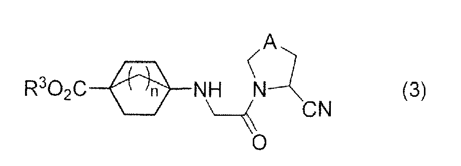 (wherein A represents CH₂, CHF or CF₂; R³ represents a C₁ to C₆ alkyl group which may have a substituent, a C₃ to C₈ cycloalkyl group which may have a substituent, an arylmethyl group which may have a substituent, an arylethyl group which may have a substituent, an aromatic hydrocarbon group which may have a substituent, an aromatic heterocyclic group which may have a substituent or an aliphatic heterocyclic group which may have a substituent; and n represents 1 or 2).
[11] A method for stabilizing an aminoacetylpyrrolidine carbonitrile derivative represented by the following general formula (1): 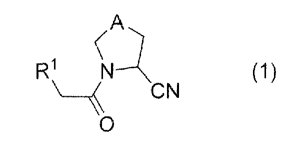 (in the formula (1), A represents CH₂, CHF or CF₂; and R¹ represents a secondary amino group which may have a substituent), wherein it comprises the step of incorporating, into the aminoacetylpyrrolidine carbonitrile derivative, the following components:
   (1) a sugar alcohol;
   (2) one or not less than 2 members selected from the group consisting of calcium carbonate, sodium citrate and sodium sulfate; and
   (3) a neutral low melting point oily and/or fatty substance.

### Effect of the Invention

According to the present invention, there can be provided a pharmaceutical composition comprising the compound represented by the foregoing general formula (1) wherein any decomposition of the compound is highly inhibited and which can easily be prepared.

### Mode for Carrying Out the Invention

The phrase "secondary amino group which may have a substituent" used in this specification means a secondary amino group which may have 1 to 5 substituents selected from the group consisting of halogen atoms, hydroxyl group, cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, amino group, mono- or di-substituted C₁ to C₆ alkylamino groups, 4- to 9-membered cyclic amino groups which may comprise 1 to 3 hetero atoms (such as nitrogen, oxygen and sulfur atoms), formylamino groups, C₁ to C₆ alkylcarbonylamino groups, C₁ to C₆ alkoxycarbonylamino groups, C₁ to C₆ alkylsulfonylamino groups and arylsulfonylamino groups which may have a substituent.

The term "secondary amino group" herein used means an aliphatic or aromatic amino group in which one hydrogen atom is linked to the nitrogen atom and examples thereof include amino groups carrying a C₁ to C₆ alkyl group linked thereto such as methylamino group or butylamino group; amino groups each carrying a C₃ to C₁₀ cyclic alkyl group such as cyclohexylamino group, adamantylamino group and bicyclo[2.2.2]octanylamino group; aromatic amino groups (such as anilyl group and pyridylamino group), and the like.
The phrase "C₁ to C₆ alkyl group which may have a substituent" used in this specification means a C₁ to C₆ alkyl group which may have 1 to 5 substituents selected from the group consisting of halogen atoms, hydroxyl group, cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, amino groups, mono- or di-substituted C₁ to C₆ alkylamino groups, 4- to 9-membered cyclic amino groups which may comprise 1 to 3 hetero atoms, formylamino group, C₁ to C₆ alkylcarbonylamino groups, C₁ to C₆ alkoxycarbonyl amino groups, C₁ to C₆ alkylsulfonylamino groups and arylsulfonylamino groups which may have a substituent.

The coverage of the term "substituent" related to the foregoing "aryloxy group which may have a substituent" or "arylsulfonylamino group which may have a substituent" may be the same as that described below in connection with the substituent for the "arylmethyl group which may have a substituent." Moreover, the coverage of the term "aryl group" may likewise be the same as that of the aryl group exemplified later in connection with the term "arylmethyl group which may have a
The same rule applies correspondingly to the following, unless otherwise expressly specified. This is also true for the phrase "arylamino group which may have a substituent" as will also be referred to below.

The term "C₁ to C₆ alkyl group" used herein means a linear or branched lower alkyl group and the specific examples thereof include methyl group, ethyl group, propyl group, 1-methylethyl group, 1-methylpropyl group, 2-methylpropyl group, 1-ethylpropyl group, 2-ethylpropyl group, butyl group, hexyl group and the like.
Incidentally, the coverage of the term "C₁ to C₆ alkyl group" used in the groups including "C₁ to C₆ alkyl group," for instance, the foregoing C₁ to C₆ alkoxy groups, C₁ to C₆ alkylcarbonyl groups, C₁ to C₆ alkoxycarbonyl groups, C₁ to C₆ alkylthio groups, mono- or di-substituted C₁ to C₆ alkylamino groups, C₁ to C₆ alkylcarbonylamino groups, C₁ to C₆ alkoxycarbonylamino groups, and C₁ to C₆ alkylsulfonylamino groups may be the same as that stated above. In the following description, this term will be used in the same meanings, unless otherwise expressly specified.

The phrase "C₃ to C₁₀ cyclic alkyl group which may have a substituent" used in this specification means a C₃ to C₁₀ cyclic alkyl group which may have 1 to 5 substituents selected from the group consisting of halogen atoms, hydroxyl group, cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, amino groups, mono- or di-substituted C₁ to C₆ alkylamino groups, 4- to 9-membered cyclic amino groups which may comprise 1 to 3 hetero atoms, formylamino group, C₁ to C₆ alkylcarbonylamino groups, C₁ to C₆ alkoxycarbonylamino groups, C₁ to C₆ alkylsulfonylamino groups and arylsulfonylamino groups which may have a substituent.

The term "C₃ to C₁₀ cyclic alkyl group" used herein means a C₃ to C₈ cycloalkyl group, a C₅ to C₁₀ bicycloalkyl group or an adamantyl group.
The term "C₃ to C₈ cycloalkyl group" used above means an alkyl group having a cycloalkyl ring and the specific examples thereof include cyclopropyl group, cyclopropylmethyl group, cyclobutyl group, cyclopentyl group, cyclohexyl group, cycloheptyl group, cyclooctyl group, and the like.
The term "C₅ to C₁₀ bicycloalkyl group" used above means an alkyl group having a bicycloalkyl ring and the specific examples thereof are bicyclopentyl group, bicyclo hexyl group, bicyclopentyl group, bicyclooctyl group, bicyclononyl group, bicyclodecyl group, and the like.

The phrase "C₂ to C₁₀ cyclic amino group which may have a substituent" herein used means a C₂ to C₁₀ cyclic amino group which may have 1 to 5 substituents selected from the group consisting of halogen atoms, hydroxyl group, cyano group, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, amino group, mono- or di-substituted C₁ to C₆ alkylamino groups, 4- to 9-membered cyclic amino groups which may comprise 1 to 3 hetero atoms, formylamino group, C₁ to C₆ alkylcarbonylamino groups, C₁ to C₆ alkoxy carbonylamino groups, C₁ to C₆ alkylsulfonylamino groups and arylsulfonylamino groups which may have a substituent.

The term "C₂ to C₁₀ cyclic amino group" used above means a cyclic amino group which comprises at least one nitrogen atom within the ring and which may have an oxygen atom and/or a sulfur atom within the ring. The examples thereof include aziridyl group, pyrrolidyl group, piperidyl group, morpholyl group, oxazolyl group, azabicycloheptyl group, azabicyclooctyl group, and the like.
The term "C₃ to C₈ cycloalkyl group which may have a substituent" used in this specification means a C₃ to C₈ cycloalkyl group which may have 1 to 5 substituents selected from the group consisting of halogen atoms, hydroxyl groups, cyano groups, C₁ to C₆ alkoxy groups, aryloxy groups which may have a substituent, C₁ to C₆ alkylcarbonyl groups, C₁ to C₆ alkoxycarbonyl groups, C₁ to C₆ alkylthio groups, amino groups, mono- or di-substituted C₁ to C₆ alkylamino groups, 4- to 9-membered cyclic amino groups which may comprise 1 to 3 hetero atoms, formylamino group, C₁ to C₆ alkylcarbonylamino groups, C₁ to C₆ alkoxy carbonylamino groups, C₁ to C₆ alkylsulfonylamino groups and arylsulfonylamino groups which may have a substituent.

The term "C₃ to C₈ cycloalkyl group" used above means an alkyl group carrying a cycloalkyl ring and the specific examples thereof include cyclopropyl group, cyclopropylmethyl group, cyclobutyl group, cyclopentyl group, cyclohexyl group, cycloheptyl group cyclooctyl group, and the like.
The term "arylmethyl group which may have a substituent" used in this specification means an arylmethyl group (for instance, phenylmethyl group, naphthylmethyl group, pyridylmethyl group, quinolylmethyl group, indolylmethyl group or the like) which may have 1 to 5 substituents selected from the group consisting of halogen atoms, C₁ to C₆ alkyl groups which may have a substituent, hydroxyl groups, cyano groups, nitro groups, C₁ to C₆ alkoxy groups which may have a substituent, aryloxy groups which may have a substituent, C₁ to C₆ alkylcarbonyl groups, C₁ to C₆ alkoxycarbonyl groups, C₁ to C₆ alkylthio groups, amino group, C₁ to C₆ alkylamino groups which may have mono- or di- substituents, arylamino groups which may have a substituent, 4- to 9-membered cyclic amino groups which may comprise 1 to 3 hetero atoms, formylamino groups, C₁ to C₆ alkylcarbonylamino groups, C₁ to C₆ alkoxycarbonylamino groups, C₁ to C₆ alkylsulfonylamino groups and arylsulfonylamino groups which may have a substituent.

The term "arylethyl group which may have a substituent" used in this specification means an arylethyl group (for instance, phenylethyl group, naphthylethyl group, pyridylethyl group, quinolylethyl group, indolylethyl group, or the like) which may have 1 to 5 substituents selected from the group consisting of halogen atoms, C₁ to C₆ alkyl groups which may have a substituent, hydroxyl groups, cyano groups, nitro groups, C₁ to C₆ alkoxy groups which may have a substituent, aryloxy groups which may have a substituent, C₁ to C₆ alkylcarbonyl groups, C₁ to C₆ alkoxycarbonyl groups, C₁ to C₆ alkylthio groups, amino groups, C₁ to C₆ alkylamino groups which may have mono- or di- substituents, arylamino groups which may have a substituent, 4- to 9-membered cyclic amino groups which may comprise 1 to 3 hetero atoms, formylamino groups, C₁ to C₆ alkylcarbonylamino groups, C₁ to C₆ alkoxycarbonylamino groups, C₁ to C₆ alkylsulfonylamino groups and arylsulfonylamino groups which may have a substituent.

The term "aromatic hydrocarbon group which may have a substituent" used in this specification means an aromatic hydrocarbon group (for instance, benzene ring, naphthalene ring or anthracene ring) which may have 1 to 5 substituents selected from the group consisting of halogen atoms, hydroxyl group, cyano group, nitro group, C₁ to C₆ alkyl groups which may have a substituent, C₁ to C₆ alkoxy groups which may have a substituent, C₁ to C₆ alkylthio groups which may have a substituent and C₁ to C₆ dialkylamino groups which may have a substituent.

The term "aromatic hetero ring which may have a substituent" used in this specification means an aromatic hetero ring (for instance, 5- or 6-membered aromatic monocyclic hetero rings which comprise 1 to 3 hetero atoms arbitrarily selected from nitrogen, oxygen and sulfur atoms; or 9- or 10-membered aromatic fused hetero rings such as pyridine ring, pyrimidine ring, pyridazine ring, triazine ring, quinoline ring, naphthyridine ring, quinazoline ring, acridine ring, pyrrole ring, furan ring, thiophene ring, imidazole ring, pyrazole ring, oxazole ring, isoxazole ring, thiazole ring, indole ring, benzofuran ring, benzothiazole ring, benzimidazole ring, and benzoxazole ring) which may have 1 to 5 substituents selected from the group consisting of halogen atoms, hydroxyl group, cyano group, nitro group, C₁ to C₆ alkyl groups, C₁ to C₆ alkoxy groups and C₁ to C₆ alkylthio groups.

The phrase "aliphatic hetero ring which may have a substituent" used in this specification means an aliphatic hetero ring (for instance, 4- to 7-membered aliphatic monocyclic hetero rings which comprise 1 to 3 hetero atoms arbitrarily selected from nitrogen, oxygen and sulfur atoms; and 9- or 10-membered aliphatic fused hetero rings such as azetidine ring, pyrrolidine ring, tetrahydrofuran ring, piperidine ring, morpholine ring, and perazine ring) which may have 1 to 5 substituents selected from the group consisting of halogen atoms, C₁ to C₆ alkyl groups, hydroxyl group, cyano group, nitro group, C₁ to C₆ alkoxy groups and C₁ to C₆ alkylthio groups.

The term "sugar alcohol" used in this specification means a polyhydric alcohol having 4 to 12 carbon atoms and the examples thereof include erythritol, xylitol, mannitol, sorbitol, maltitol, and lactitol.
As such sugar alcohol, preferably used herein include those having a critical relative humidity as determined at 37°C of not less than 70% and further preferably used herein are those having a critical relative humidity as determined at 37°C of not less than 85%. In this respect, the term "critical relative humidity" means the relative humidity observed when the water content of a specific sugar alcohol is abruptly increased and has been well known as a parameter for the indication of hygroscopicity thereof. For instance, the critical relative humidity can be determined by introducing samples (obtained after drying at 80°C overnight) of about 1 g each into weighing bottles, placing them in thermo-hygrostats which have been set at the following relative humidity (RH) values: 37°C/40%RH, 37°C/45%RH, 37°C/55%RH, 37°C/65%RH, 37°C/70%RH, 37°C/75%RH, 37°C/80%RH, 37°C/85%RH, 37°C/90%RH, 37°C/95%RH and 37°C/98%RH, respectively (all of the chambers are of the same type: ETAC FX201P), allowing them to stand under such conditions over 72 hours, calculating the increase in the moisture content of each sample at this stage, and determining the lowest humidity at which an increase in the moisture content is observed, the resulting lowest humidity being defined to be the critical relative humidity of the sugar alcohol thus tested. In this respect, a substance having a high critical relative humidity would show a low hygroscopicity. The examples of such sugar alcohols having a critical relative humidity, as determined at 37°C, of not less than 70% are erythritol, xylitol, mannitol, maltitol, lactitol, and the like. Moreover, the examples of sugar alcohols having a critical relative humidity, as determined at 37°C, of not less than 85% are mannitol and erythritol. Mannitol has a critical relative humidity of not less than 98% and accordingly, it can particularly preferably be used in the present invention. In addition, the foregoing sugar alcohols may be used in combination.

The term "neutral low melting point oily and/or fatty substance" used in this specification means a neutral substance which shows an oily and/or fatty appearance, which usually has a melting point ranging from 20 to 90°C and preferably 20 to 60°C and which is solid at ordinary temperature. This substance may be any one, inasmuch as it never adversely affect the compound represented by the foregoing general formula (1) and the examples thereof include hydrocarbons, higher alcohols, fatty acid esters of polyhydric alcohols, higher alcohol ethers of polyhydric alcohols, polymers of alkylene oxides, and the like. Preferably used herein are higher alcohols or polymers of alkylene oxides and more preferably used herein are higher alcohols. The examples of the hydrocarbons usable in the present invention are n-alkanes having 17 to 50 carbon atoms such as n-heptadecane, n-octadecane, n-nonadecane, n-eicosane, n-heneicosane, n-docosane, n-tricosane, n-tetra- cosane, n-pentacosane, n-triacontane, n-pentatriacontane, n-tetracontane and n-pentacontane; and mixture thereof (such as petrolatum, paraffin waxes and microcrystalline waxes).

The examples of the foregoing higher alcohols capable of being used in the present invention include lauryl alcohol, myristyl alcohol, cetyl alcohol and stearyl alcohol. Preferably used herein as such higher alcohols include cetyl alcohol and stearyl alcohol, with stearyl alcohol being further preferably used herein.

The examples of the foregoing fatty acid esters of polyhydric alcohols usable in the present invention are esters between alcohols having not less than 2 hydroxyl groups in the molecule (for instance, alkylene glycols such as ethylene glycol and propylene glycol; polyalkylene glycols such as polyethylene glycol, polypropylene glycol and copolymers thereof saccharides such as sorbitol and sucrose; intra-molecularly dehydrated compounds of sorbitol such as 1,5-sorbitan, 1,4-sorbitol and 3,6-sorbitan; glycerin; diethanolamine; pentaerythritol, and the like), and fatty acids (such as acetic acid, propionic acid, butyric acid, pelargonic acid, capric acid, undecylic acid, lauric acid, tridecylic acid, myristic acid, pentadecylic acid, palmitic acid, heptadecylic acid, stearic acid, nonadecanoic acid, undecylenic acid, oleic acid, elaidic acid, sorbic acid, linoleic acid, linolenic acid, arachidonic acid, stearolic acid, and the like),

for instance, sorbitan fatty acid esters having a molecular weight ranging from 400 to 900 such as sorbitan monostearate, sorbitan tristearate, sorbitan monooleate, sorbitan sesquioleate sorbitan monopalmitate, and the like; polyoxyalkylene sorbitan fatty acid esters each having a molecular weight ranging from 1000 to 1500 such as polyoxyethylene sorbitan tristearate, polyoxyethylene sorbitan monooleate, polyoxyethylene sorbitan tripalmitate, and the like; polyoxyalkylene sorbitol fatty acid esters such as polyoxyethylene sorbitol hexastearate, polyoxyethylene sorbitol hexaoleate, polyoxyethylene sorbitol tristearate, polyoxyethylene sorbitol tetralaurate, and the like; polyoxyalkylene sorbitol bees wax derivatives such as polyoxyethylene sorbitol bees wax derivatives, and the like; polyoxyalkylene lanolin derivatives such as polyoxyethylene lanolin derivatives, and the like; alkylene glycol fatty acid esters, for instance, propylene glycol fatty acid esters each having a molecular weight ranging from 200 to 700 such as propylene glycol monopalmitate, propylene glycol monostearate, propylene glycol dilaurate, propylene glycol dimyristate, propylene glycol dipalmitate, propylene glycol distearate, and the like; ethylene glycol fatty acid esters each having a molecular weight ranging from 500 to 1200 such as ethylene glycol monolaurate, ethylene glycol palmitate, ethylene glycol margarate, ethylene glycol stearate, ethylene glycol dilaurate, ethylene glycol dimyristate, ethylene glycol dipalmitate

, ethylene glycol dimargarate, and the like; polyoxyalkylene castor oil derivatives having a molecular weight ranging from 3500 to 4000 such as polyoxyethylene castor oil derivatives, and the like; polyoxyalkylene fatty acid esters having a molecular weight ranging from 1900 to 2200 such as polyoxyethylene stearate, polyoxyethylene oleate, polyoxyethylene palmitate polyoxyethylene linoleate, and the like; glycerin monofatty acid esters having a molecular weight ranging from 300 to 600 such as glycerin monoacetate, glycerin monopropionate, glycerin monostearate, glycerin monooleate, glycerin monopalmitate, glycerin monolinoleate, and the like; and sucrose fatty acid esters having a molecular weight ranging from 400 to 1300 such as sucrose monolaurate, sucrose monomyristate, sucrose monopalmitate, sucrose monostearate, sucrose trimyristate, sucrose tripalmitate sucrose tristearate, and the like.

The examples of the foregoing higher alcohol ethers of polyhydric alcohols, which can be used in the present invention, include ethers of polyhydric alcohols (such as those specified above in connection with the alcoholic components of the foregoing fatty acid esters of polyhydric alcohols) with higher fatty acid alcohols (such as cetyl alcohol, stearyl alcohol, oleyl alcohol, octyl alcohol decyl alcohol, and the like), for instance, polyoxyethylene higher alcohol ethers such as polyoxyethylene lauryl alcohol ether, polyoxyethylene cetyl alcohol ether, polyoxyethylene stearyl alcohol ether, polyoxyethylene oleyl alcohol ether, polyoxyethylene octyl alcohol ether, polyoxyethylene decyl alcohol ether, and the like; and polyoxypropylene polyoxyethylene higher alcohol ethers such as polyoxypropylene polyoxyethylene cetyl alcohol ether, polyoxypropylene polyoxyethylene stearyl alcohol ether, polyoxypropylene polyoxyethylene oleyl alcohol ether, polyoxypropylene polyoxyethylene octyl alcohol ether, polyoxypropylene polyoxyethylene lauryl alcohol ether, and the like.
The examples of the aforementioned polymers of alkylene oxides usable in the present invention include those having a molecular weight ranging from 1,000 to 10,000 such as polyethylene glycol 4000 and polyethylene glycol 6000. In the present invention, these neutral low melting point oily and/or fatty substances may be used alone or in any combination of at least two thereof.

In the pharmaceutical composition according to the present invention, there can optionally or depending on necessity, be incorporated into the composition, other additives such as excipients, binders, disintegrating agents, lubricants, film-coating base materials, sugar-coating base materials, plasticizers, coloring agents, taste-corrective agents, odor-corrective agents and perfumes, inasmuch as they do not adversely affect the intended effects of the present invention.
In the pharmaceutical composition according to the present invention, excipients other than the foregoing sugar alcohols can additionally be used in an amount which does not impair the intended stabilization effect. The examples of such excipients, herein usable, other than the sugar alcohols include crystalline cellulose, glucose, fructose, maltose, lactose, isomerized lactose, reduced lactose, sucrose, corn starch, potato starch, wheat starch, rice starch, crystalline cellulose, talc, silicic acid anhydride, anhydrous calcium phosphate, precipitated calcium carbonate, calcium silicate, and the like.

The aforementioned binders usable in the present invention are, for instance, hydroxypropyl cellulose, Hypromellose, Povidone, polyvinyl pyrrolidone, methyl cellulose, polyvinyl alcohol, carboxymethyl cellulose, partially pregelatinized starch, pregelatinized starch, sodium alginate, pullulan, Acacia powder, gelatin, dextrin, and the like.
The foregoing disintegrating agents usable in the present invention include, for instance, low substituted hydroxypropylcellulose, carmellose, carmellose calcium, sodium carboxymethyl starch, sodium croscarmellose, crospovidone, hydroxypropyl starch, corn starch, partially pregelatinized starch, and the like. The foregoing lubricants which can be used herein include, for instance, magnesium stearate, calcium stearate, sucrose fatty acid esters, and the like.

The foregoing film-coating base materials usable in the present invention include, for instance, methyl cellulose, ethyl cellulose, hydroxypropyl cellulose, Hypromellose, polyvinyl pyrrolidone, and the like.
The foregoing sugar-coating base materials usable in the present invention include, for instance, sucrose, trehalose, lactose, mannitol, powdered reduced maltose starch syrup, and the like.
In the present invention, when carrying out the film-coating or the sugar-coating operations, additives such as excipients, plasticizers, and/or coloring agents can be incorporated into the coatings, depending on necessity.

The examples of such excipients are talc, calcium carbonate, titanium oxide, and the like. The plasticizers usable herein are, for instance, Macrogol 6000, Copolyvidone, triethyl citrate, and the like. The examples of the foregoing coloring agents include titanium oxide, Food Yellow No. 5, Food Blue No. 2, red ferric oxide, yellow ferric oxide, and the like. The taste-corrective agents which can be used in the present invention are, for instance, white soft sugar, sorbitol, xylitol, citric acid, ascorbic acid, tartaric acid, malic acid, aspartame, acesulfame potassium, thaumatin, saccharin sodium, glycyrrhizin dipotassium, sodium glutamate, disodium inosinate, disodium 5'-guanlate, and the like. The odor-corrective agents usable in the present invention include, for instance, trehalose, malic acid, maltose, potassium gluconate, anise essential oil, vanilla essential oil and cardamon essential oil. The examples of perfumes usable in the present invention include lemon oil, orange oil, mentha oil, menthol, and the like.

The pharmaceutical composition according to the present invention can be prepared by adding the aforementioned neutral low melting point oily and/or fatty substance to the granules containing the compound represented by the foregoing general formula (1), and then molding the resulting mixture. These ingredients can be blended according to the usual mixing techniques currently used in the drug-manufacturing techniques such as mixing techniques, kneading techniques, sieving techniques and stirring techniques. For instance, the neutral low melting point oily and/or fatty substance may directly be added to and admixed with the granules containing the compound represented by the general formula (1). Alternatively, the neutral low melting point oily and/or fatty substance may first be dissolved in a proper solvent and then the resulting solution may uniformly be admixed with the granules containing the compound of formula (1) by way of mixing with the granules or spraying the solution thereon. The examples of solvents, appropriately used when adding the neutral low melting point oily and/or fatty compound in the form of a liquid, include those which do not adversely affect the compound of formula (1) such as water, dimethylformamide, acetone, ethanol, propyl alcohol, isopropyl alcohol, butyl alcohol, methylene chloride, trichloroethane, and the like. These solvents may be used alone or in any combination of at least two of them. Preferably used in the present invention are, for instance, water, ethanol, or mixture containing water and ethanol.

The pharmaceutical composition according to the present invention may be formed into any dosage form such as granules, fine granules, uncoated tablets, film-coated tablets, sugar-coated tablets, chewable tablets, capsules, and the like. The pharmaceutical composition of the present invention may be prepared according to any known method selected while taking into consideration each particular dosage form of the composition. These pharmaceutical compositions can suitably be prepared according to the usual techniques disclosed in, for instance, A handbook for Granulation (edited by the Association of Powder Process Industry and Engineering, JAPAN, published by Ohm Corporation); Formulation and Design of Orally Administrable Pharmaceutical Preparations (edited by HASHIDA Mitsuru, the professor of a faculty of pharmaceutical research, a graduated school of Kyoto University, published by Yakugyo Jiho-Sha); and Techniques for Compression-Molding of Powder (edited by the Section of Powder Engineering Designing of Pharmaceutical Preparations and Particles, published by The Nikkan Kogyo Shimbun Ltd.). For instance, there are admixed together a compound represented by the foregoing general formula (1), calcium carbonate, an excipient, and a binder, the resulting mixture is granulated and then sieved to control the particle size of the granulates and to thus obtain sized granulates. Thereafter, there are added, to the resulting particle size-controlled granulates, higher alcohols, a disintegrating agent, and a lubricant and the resulting mixture is then formed into tablets to thus give uncoated tablets. Film-coating is applied to the resulting uncoated tablets to thus obtain film-coated tablets.

### Examples

Then the present invention will be described with reference to the following Examples and Test Examples, but the scope of the present invention is by no means limited to those specific Examples and Test Examples at all. In this respect, the compound 1 used in Examples can be prepared according to the method disclosed in WO 2005/075421.

### Example 1

Using a fluidized bed granulator (FBG-1 available from Freund Industry Co., Ltd.) and according to the following formulation, mannitol and low substituted hydroxypropylcellulose were admixed with (2S,4S)-1-[2-[(4-ethoxycarbonylbicyclo[2.2.2]oct-1-yl)amino] acetyl]-4-fluoropyrrolidine-2-carbonitrile (hereunder simply referred to as "compound 1"), which had been milled using a hummer-type milling apparatus (K11G-1S available from Fuji Powdal Co., Ltd.), followed by the granulation of the resulting blend powders by spraying an aqueous solution of hydroxypropyl cellulose as a binder liquid on the powders, the subsequent drying and particle size-controlling of the granules. Thereafter, magnesium stearate was added to the granules, followed by the blending of the resulting mixture with the use of a V-shaped mixer (15L, available from Nippon Yakugyo Kikai K. K.) and the subsequent forming of the resulting blend into tablets each having a weight of 150 mg and a thickness of 3.4 mm using a tablet machine (HT-A-P-18-SSII available from Hata Iron Works Co., Ltd.) equipped with R-face punches of 9 mm radius and dies of 7.5 mm in diameter under the compression force ranging from 530 to 590 kg. Further a water-based coating solution containing Hypromellose was applied onto the tablets using a coating machine (HCT-MINI available from Freund Industry Co., Ltd.) and then the resulting coated tablets were polished through the use of carnauba wax.

### Example 2

The same procedures used in Example 1 were repeated except that lactose was substituted for mannitol and the pressure during the tabletting operations was changed to 430 to 490 kg to thus form tablets.

[Table 1]

**Table 1**

| **Component** | **Example 1** | **Example 2** |
|---|---|---|
| Compound represented by the general formula (1) | 5 | 5 |
| Lactose | -- | 133.5 |
| Mannitol | 131.5 | -- |
| Hydroxypropyl Cellulose | 6 | 6 |
| Low substituted hydroxypropylcellulose | 6 | 4 |
| Magnesium Stearate | 1.5 | 1.5 |
| Subtotal Amount (mg) | 150 | 150 |
| Hypromellose | 4 | 4 |
| Carnauba Wax | Trace amt. | Trace amt. |
| Total Amount (mg) | 154 | 154 |

### Test Example 1

The tablets prepared in Example 1 and Example 2 were put in glass bottles, the glass bottles were hermetically sealed and stored at 40°C for 4 weeks under such conditions. Thereafter, the amounts of the decomposition products of the compound 1 generated during the storage were determined by liquid chromatography and the contents thus determined were expressed in terms of the percentages relative to the content of the compound 1. In this respect, the detection limit in the quantitative analysis for determining the contents of the decomposition products was 0.05% and therefore, each result did not include the amount of decomposition products less than the detection limit in the quantitative analysis.

### Liquid Chromatography Test Conditions :

Column: Silica gel (5 µ m each) for liquid chromatography, which had preliminarily been subjected to octadecyl silylation was charged into a stainless tube having an inner diameter of 4.6 mm and a length of 5 mm and another stainless tube having an inner diameter of 4.6 mm and a length of 150 mm and the former stainless tube was used as a guard column and the latter stainless tubes was used as a separation column, respectively (Inertsil ODS-3 and Inertsil ODS-3V, available from GL Science Company).
Liquid A: Sodium 1-octanesulfonate (2.16 g) was dissolved in diluted phosphoric acid (1→ 1000) and then the total volume thereof was precisely adjusted to 1000 mL.
Liquid B: Acetonitrile for liquid chromatography.
Feed Liquid: The concentration gradient was controlled by properly changing the mixing ratio of the liquid A and the liquid B.
Detector: Ultraviolet Absorption Spectrometer (Wavelength of Measurement: 210 nm).

[Table 2]

| **Table 2: Results of Stability Test** | | |
|---|---|---|
| **Item to be Determined** | **Example 1** | **Example 2** |
| Content (%) of decomposition products observed after storing at 40°C for 4 weeks | 0.09 | 0.44 |

As will be clear from the data shown in the foregoing Table 2, the tablets obtained using mannitol in Example 1 were found to be more stable as compared with those prepared using lactose in Example 2.

### Example 3

Using a fluidized bed granulator (FBG-1 available from Freund Industry Co., Ltd.) and according to the following formulation, mannitol, low substituted hydroxypropylcellulose and calcium carbonate were admixed with the compound 1, which had been milled using a hummer-type milling apparatus (K11G-1S available from Fuji Powdal Co., Ltd.), followed by the granulation of the resulting blend powders by spraying an aqueous solution of hydroxypropyl cellulose as a binder liquid on the powders, the subsequent drying and particle size-controlling of the granules. Thereafter, magnesium stearate was added to the granules followed by the blending of the resulting mixture while making use of a V-shaped mixer (15L, available from Nippon Yakugyo Kikai K. K.) and the subsequent forming of the mixture into tablets each having a weight of 150 mg and a thickness of 3.4 mm using a tablet machine (HT-AP-18-SSII available from Hata Iron Works Co., Ltd.) equipped with R-face punches of 9 mm radius and dies of 7.5 mm in diameter under the compression force ranging from 580 to 640 kg.

### Example 4

The same procedures used in Example 3 were repeated except that sodium citrate was substituted for calcium carbonate and the pressure during the tabletting operations was changed to 590 to 640 kg to thus form tablets.

### Example 5

The same procedures used in Example 3 were repeated except that sodium sulfate was used in place of calcium carbonate and the pressure used during the tabletting step was changed to 570 to 620 kg to thus form tablets.

### Example 6

Using a fluidized bed granulator (FBG-1 available from Freund Industry Co., Ltd.) and according to the following formulation, mannitol and low substituted hydroxypropylcellulose were admixed with the compound 1, which had preliminarily been milled using a hummer-type milling apparatus (K11G-1S available from Fuji Powdal Co., Ltd.), followed by the granulation of the resulting blend powders by spraying an aqueous solution of hydroxypropyl cellulose as a binder liquid on the powders, the subsequent drying and particle size-controlling of the granules. Thereafter, magnesium stearate was added to the granules, followed by the blending of the resulting mixture while making use of a V-shaped mixer (15L, available from Nippon Yakugyo Kikai K. K.) and the subsequent forming of the mixture into tablets each having a weight of 150 mg and a thickness of 3.4 mm using a tablet machine (HT-AP-18-SSII available from Hata Iron Works Co., Ltd.) equipped with R-face punches of 9 mm radius and dies of 7.5 mm in diameter under the compression force ranging from 580 to 600 kg.

[Table 3]

| **Table 3** | | | | |
|---|---|---|---|---|
| **Component** | **Ex. 3** | **Ex. 4** | **Ex. 5** | **Ex. 6** |
| Compound represented by the general formula (1) | 1 | 1 | 1 | 1 |
| Mannitol | 128 | 128 | 128 | 135.5 |
| Low substituted hydroxypropylcellulose | 6 | 6 | 6 | 6 |
| Calcium carbonate | 7.5 | -- | -- | -- |
| Sodium citrate | -- | 7.5 | -- | -- |
| Sodium sulfate | -- | -- | 7.5 | -- |
| Hydroxypropyl cellulose | 6 | 6 | 6 | 6 |
| Magnesium stearate | 1.5 | 1.5 | 1.5 | 1.5 |
| Total Amount (mg) | 150 | 150 | 150 | 150 |

### Test Example 2

Stability-determining tests were carried out by repeating the same procedures used in Test Example 1 except that the tablets prepared in Examples 3 to 6 were charged in glass bottles. The liquid chromatography procedures were likewise carried out under the same test conditions used in Test Example 1.

[Table 4]

| **Table 4: Results of the foregoing stability tests** | | | | |
|---|---|---|---|---|
| **Item to be examined** | **Ex. 3** | **Ex. 4** | **Ex. 5** | **Ex. 6** |
| Content (%) of decomposition products observed after storing at 40°C for 4 weeks | 0.37 | 0.71 | 0.52 | 0.89 |

In the tablets prepared in Examples 3 to 6, the compound 1 was used in an amount of 1 mg and therefore, the contents of the decomposition products were greater than those observed for the tablets each containing 5 mg of the compound 1 (as will be clear when comparing them with the result observed for the tablet prepared in Example 1 and Example 7 as will be described below). However, the data listed in the foregoing Table 4 clearly indicate that the tablets prepared in Example 3 (calcium carbonate was used as the stabilizer), Example 4 (sodium citrate was used as the stabilizer) and Example 5 (sodium sulfate was used as the stabilizer) were found to be stable even though the tablets each contained 1 mg of the compound 1. More specifically, the contents of the decomposed products observed for these tablets were found to be low and the tablets were stable as compared with those prepared in Example 6 which did not use any combination of stabilizers (i.e., the combination of mannitol with, for instance, calcium carbonate) and used only mannitol as the stabilizer.

### Example 7

Using a fluidized bed granulator (FBG-1 available from Freund Industry Co., Ltd.) and according to the following formulation, mannitol was admixed with the compound 1, which had been milled using a hummer-type milling apparatus (K11G-1S available from Fuji Powdal Co., Ltd.), followed by the granulation of the resulting blend powders by spraying an aqueous solution of hydroxypropyl cellulose on the blend powder, the subsequent drying of the blend granules, the spraying of a liquid obtained by dissolving stearyl alcohol in a mixed solvent of water and ethanol on the granulated mixture, the drying and particle size-controlling of the granules. Thereafter, low substituted hydroxypropylcellulose was added to the granules, followed by the blending of them with the use of a V-shaped mixer (15L, available from Nippon Yakugyo Kikai K. K.), the subsequent addition of magnesium stearate to the resulting blend, the blending of the mixture in a V-shaped mixer (15L, available from Nippon Yakugyo Kikai K. K.) and the subsequent forming of the resulting blend into tablets each having a weight of 150 mg and a thickness of 3.4 mm using a tablet machine (HT-AP-18-SSII available from Hata Iron Works Co., Ltd.) equipped with R-face punches of 9 mm radius and dies of 7.5 mm in diameter under the compression force ranging from 540 to 570 kg. Further a water-based coating solution containing Hypromellose was applied onto the tablets using a coating machine (HCT-MINI available from Freund Industry Co., Ltd.) and then the resulting coated tablets were polished through the use of carnauba wax.

[Table 5]

| **Table 5** | |
|---|---|
| **Component** | **Ex. 7** |
| Compound represented by the general formula (1) | 5 |
| Mannitol | 133.2 |
| Hydroxypropyl cellulose | 6 |
| Stearyl alcohol | 0.3 |
| Low substituted hydroxypropylcellulose | 4 |
| Magnesium stearate | 1.5 |
| **Subtotal Amount (mg)** | 150 |
| Hypromellose | 4 |
| Carnauba wax | Trace amt. |
| **Total Amt. (mg)** | **154** |

### Test Example 3

The tablets prepared in Example 7 were charged into glass bottles, the glass bottles were hermetically sealed and they were stored at 40°C for 8 weeks under such sealed conditions. Thereafter, the amounts of the decomposition products of the compound 1 generated during the storage were determined by the liquid chromatography technique and the contents thus determined were expressed in terms of the percentages relative to the content of the compound 1. In this respect, the detection limit in the quantitative analysis for determining the contents of the decomposition products was 0.05% and therefore, each result did not include the amount of decomposition products less than the detection limit in the quantitative analysis. The liquid chromatography procedures were likewise carried out under the same test conditions used in Test Example 1.

[Table 6]

| **Table 6: Result of the Stability Test** | |
|---|---|
| **Item to be Examined** | **Ex. 7** |
| Content (%) of decomposition products observed after storing at 40°C for 8 weeks | Not detected |

As shown in Table 6, there was not detected any decomposition product for the tablet obtained in Example 7 while using mannitol and stearyl alcohol as stabilizers even after storing the same over 8 weeks. More specifically, the tablet of Example 7 was found to be significantly stable as compared with the tablet prepared in Example 2 in which lactose was substituted for the sugar alcohol and which was free of any calcium carbonate and stearyl alcohol.

### Example 8 (Example of the present invention)

Using a fluidized bed granulator (FBG-1 available from Freund Industry Co., Ltd.) and according to the following formulation, mannitol and calcium carbonate were admixed with the compound 1, which had been milled using a hummer-type milling apparatus (K11G-1S available from Fuji Powdal Co., Ltd.), followed by the granulation of the resulting blend powders by spraying an aqueous solution of hydroxypropyl cellulose on the blend powders, the subsequent drying of the granulated particles, the spraying, on the resulting granulated particles, of a liquid obtained by dissolving stearyl alcohol in a mixed solvent of water and ethanol, the drying and particle size-controlling of the particles. Thereafter, low substituted hydroxypropylcellulose was added to the particles, followed by the blending of them with the use of a V-shaped mixer (15L, available from Nippon Yakugyo Kikai K. K.), the addition of magnesium stearate to the resulting blend, the blending of the mixture in a V-shaped mixer (15L, available from Nippon Yakugyo Kikai K. K.) and the subsequent forming of the resulting blend into tablets each having a weight of 150.5 mg and a thickness of 3.4 mm using a tablet machine (HT-AP-18-SSII available from Hata Iron Works Co., Ltd.) equipped with R-face punches of 9 mm radius and dies of 7.5 mm in diameter under the compression force ranging from 540 to 590 kg. Further, a water-based coating solution containing Hypromellose was applied onto the tablets using a coating machine (HCT-MINI available from Freund Industry Co., Ltd.) and then the resulting coated tablets were polished through the use of carnauba wax.

### Example 9 (Example of the present invention)

The same procedures used in Example 8 were repeated except that cetyl alcohol was substituted for stearyl alcohol and that the pressure during the tabletting step was changed to 480 to 520 kg to thus form tablets.

[Table 7]

| **Table 7** | | |
|---|---|---|
| **Component** | **Ex.8** | **Ex.9** |
| Compound represented by the general formula (1) | 5 | 5 |
| Mannitol | 126 | 125.5 |
| Calcium carbonate | 7.5 | 7.5 |
| Hydroxypropyl cellulose | 6 | 6 |
| Stearyl alcohol | 0.5 | -- |
| Cetyl alcohol | -- | 0.5 |
| Low substituted hydroxypropylcellulose | 4 | 4 |
| Magnesium stearate | 1.5 | 1.5 |
| **Subtotal Amount (mg)** | **150.5** | **150** |
| Hypromellose | 4 | 4 |
| Carnauba wax | Trace amt. | Trace amt. |
| **Total Amount (mg)** | **154.5** | **154** |

### Test Example 4

Stability-determining tests were carried out by repeating the same procedures used in Test Example 3 except that the tablets prepared in Examples 8 and 9 were charged in glass bottles. The liquid chromatography procedures were likewise carried out under the same test conditions used in Test Example 1.

[Table 8]

| **Table 8: Results of Stability Tests** | | |
|---|---|---|
| **Item to be Tested** | **Ex. 8** | **Ex. 9** |
| Content (%) of decomposition products observed after storing at 40°C for 8 weeks | Not Detected | Not Detected |

As will be clear from the test results shown in Table 8, the tablets prepared in Example 8 in which calcium carbonate and stearyl alcohol were used as the stabilizer and those obtained in Example 9 in which calcium carbonate and cetyl alcohol were used as the stabilizer are found to have low contents of decomposed products and to be stable as compared with those obtained in Example 14 which will be described below, which was free of any sugar alcohol.

### Example 10 (Example of the present invention)

Using a fluidized bed granulator (FBG-1 available from Freund Industry Co., Ltd.) and according to the following formulation, mannitol and calcium carbonate were admixed with the compound 1, which had been milled using a hummer-type milling apparatus(K11G-1S available from Fuji Powdal Co., Ltd.), followed by the granulation of the resulting blend powders by spraying an aqueous solution of hydroxypropyl cellulose on the blend powders and the subsequent drying and particle size-controlling of the granules. Then, stearyl alcohol which had been pulverized using a pulverizer (ND-30S available from Okada Seiko Co., Ltd.) was added to and mixed with the particle size-controlled particles in a V-shaped mixer (15L, available from Nippon Yakugyo Kikai K. K.), followed by the addition of low substituted hydroxypropylcellulose to the mixture, the blending thereof in a V-shaped mixer (15L, available from Nippon Yakugyo Kikai K. K.), the addition of magnesium stearate to the resulting blend, the blending of the mixture in a V-shaped mixer (15L, available from Nippon Yakugyo Kikai K. K.) and the subsequent forming of the resulting blend into tablets each having a weight of 150 mg and a thickness of 3.4 mm using a tablet machine (HT-AP-18-SSII available from Hata Iron Works Co., Ltd.) equipped with R-face punches of 9 mm radius and dies of 7.5 mm in diameter under the compression force ranging from 470 to 530 kg. Further, a water-based coating solution containing Hypromellose was applied onto the tablets using a coating machine (HCT-MINI available from Freund Industry Co., Ltd.) and then the resulting coated tablets were polished through the use of carnauba wax.

### Example 11 (Example of the present invention)

The same procedures used in Example 10 were repeated except that xylitol was substituted for mannitol and that the pressure during the tabletting step was changed to 330 to 360 kg to thus form tablets.

### Example 12 (Example of the present invention)

The same procedures used in Example 10 were repeated except that erythritol was substituted for mannitol and that the pressure during the tabletting step was changed to 680 to 740 kg to thus form tablets.

### Example 13 (Example of the present invention)

The same procedures used in Example 10 were repeated except that sorbitol was substituted for mannitol and that the pressure during the tabletting step was changed to 300 to 340 kg to thus form tablets.

### Example 14

The same procedures used in Example 10 were repeated except that lactose was substituted for mannitol and that the pressure during the tabletting step was changed to 400 to 430 kg to thus form tablets.

[Table 9]

| **Table 9** | | | | | |
|---|---|---|---|---|---|
| **Component** | **Ex.10** | **Ex.11** | **Ex. 12** | **Ex. 13** | **Ex. 14** |
| Compound of the general formula (1) | 5 | 5 | 5 | 5 | 5 |
| Mannitol | 125.7 | -- | -- | -- | -- |
| Xylitol | -- | 125.7 | -- | -- | -- |
| Erythritol | -- | -- | 125.7 | -- | -- |
| Sorbitol | -- | -- | -- | 125.7 -- | |
| Lactose | -- | -- | -- | -- | 125.7 |
| Calcium carbonate | 7.5 | 7.5 | 7.5 | 7.5 | 7.5 |
| Hydroxypropyl cellulose | 6 | 6 | 6 | 6 | 6 |
| Stearyl alcohol | 0.3 | 0.3 | 0.3 | 0.3 | 0.3 |
| Low substituted hydroxypropylcellulose | 4 | 4 | 4 | 4 | 4 |
| Magnesium stearate | 1.5 | 1.5 | 1.5 | 1.5 | 1.5 |
| **Subtotal Amount (mg)** | **150** | **150** | **150** | **150** | **150** |
| Hypromellose | 4 | 4 | 4 | 4 | 4 |
| Carnauba wax | Trace Amount | | | | |
| **Total Amount (mg)** | **154** | **154** | **154** | **154** | **154** |

### Test Example 5

Stability-determining tests were carried out by repeating the same procedures used in Test Example 1 except that the tablets prepared in Examples 10 to 14 were charged in glass bottles. The liquid chromatography procedures were likewise carried out under the same test conditions used in Test Example 1.

[Table 10]

| **Table 10: Results of Stability Tests** | | | | | |
|---|---|---|---|---|---|
| **Item to be Tested** | **Ex. 10** | **Ex. 11** | **Ex. 12** | **Ex. 13** | **Ex. 14** |
| Content (%) of decomposition products observed after storing at 40°C for 4 weeks | Not Detected | | | 0.06 | 0.13 |

The results described above clearly indicate that the tablets obtained in Example 10, in which mannitol, calcium carbonate and stearyl alcohol are used as the stabilizer, are found to have a low content of decomposition products and to be quite stable as compared with those obtained in Example 2 in which only lactose is used and which are free of any stabilizer. The tablets obtained in Examples 11 to 13 which made use of sugar alcohols other than mannitol were likewise found to be stable. On the other hand, the tablets obtained in Example 14 in which lactose was used in place of a sugar alcohol showed low contents of decomposition products as compared with those obtained in Example 2 in which only lactose was used and which were free of any stabilizer, but the contents of decomposition products observed for the former were significantly high when comparing them with those observed for the tablets obtained in Examples 10 to 13.

As has been described above in detail, in the pharmaceutical composition containing the aminoacetylpyrrolidine carbonitrile derivative prepared according to the production method of the present invention, it was found that the decomposition of the derivative as an effective component of the pharmaceutical composition was considerably inhibited. More specifically, the present invention can thus provide a pharmaceutical composition in which the decomposition of the effective component thereof is highly controlled and which can easily be prepared by the use of a sugar alcohol, calcium carbonate and a neutral low melting point oily and/or fatty substance such as stearyl alcohol and the like in combination. Moreover, when using a sugar alcohol having a critical relative humidity, as determined at 37°C, of not less than 70%, an excellent effect (effect of controlling the decomposition of an effective component) can be obtained since any contaminant was not detected at all in Examples 10 to 12. In addition, the stabilization effect can be maintained in the pharmaceutical compositions prepared in Examples 10 and 12 even under such severe conditions that the tablets were stored in the open air. Accordingly, it would be recognized that the stabilization effect is further improved through the use of a sugar alcohol having a critical relative humidity, as determined at 37°C, of not less than 85%. In this respect, however, the practically measured critical relative humidity values are 65% for sorbitol, 70% for xylitol, 85% for erythritol and not less than 98% for mannitol.

On the other hand, when using stearic acid as an acidic low melting point oily and/or fatty substance instead of a neutral low melting point oily and/or fatty substance, it was confirmed that the resulting pharmaceutical composition had a high content of decomposition products even before the composition was formed into tablets and that the composition was stabilized through the use of a neutral low melting point oily and/or fatty substance as a stabilizer.

### Industrial Applicability

According to the present invention, there can be provided a pharmaceutical composition containing the compound represented by the general formula (1), wherein the decomposition of the compound is highly inhibited and which can easily be prepared. Therefore, the present invention is industrially useful.

## Claims

1. A stabilized pharmaceutical composition comprising an amino acetylpyrrolidine carbonitrile derivative represented by the following general formula (1): 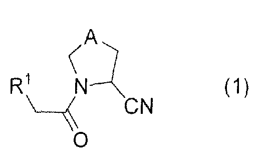 (in the formula (1), A represents CH₂, CHF or CF₂; and
R¹ represents a secondary amino group which may have a substituent), wherein it comprises:
(1) the compound represented by the foregoing general formula (1);
(2) a sugar alcohol;
(3) one or not less than 2 compounds selected from the group consisting of calcium carbonate, sodium citrate and sodium sulfate; and
(4) a neutral low melting point oily and/or fatty substance.

2. The composition as set forth in claim 1, wherein the sugar alcohol has a critical relative humidity of not less than 70%.

3. The composition as set forth in claim 1, wherein the sugar alcohol has a critical relative humidity of not less than 85%.

4. The composition as set forth in claim 1, wherein the sugar alcohol is at least one member selected from the group consisting of mannitol, xylitol, erythritol, maltitol, lactitol and sorbitol.

5. The composition as set forth in claim 1, wherein the sugar alcohol is mannitol or erythritol.

6. The composition as set forth in any one of claims 1 to 5, wherein the neutral low melting point oily and/or fatty substance is a hydrocarbon, a higher alcohol, a fatty acid ester of a polyhydric alcohol, a higher alcohol ether of a polyhydric alcohol or a polymer of an alkylene oxide.

7. The composition as set forth in claim 6, wherein the neutral low melting point oily and/or fatty substance is a higher alcohol.

8. The composition as set forth in claim 7, wherein the higher alcohol is stearyl alcohol or cetyl alcohol.

9. The composition as set forth in any one of claims 1 to 8, wherein the substituent R¹ of the compound represented by the general formula (1) is a secondary amino group represented by the following general formula (2):
R²-NH- (2)
(wherein R² represents a C₁ to C₆ alkyl group which may have a substituent, a C₃ to C₁₀ cyclic alkyl group which may have a substituent or a C₂ to C₁₀ cyclic amino group which may have a substituent).

10. The composition as set forth in any one of claims 1 to 9, wherein the compound represented by the general formula (1) is an aminoacetylpyrrolidine carbonitrile derivative represented by the following general formula (3): 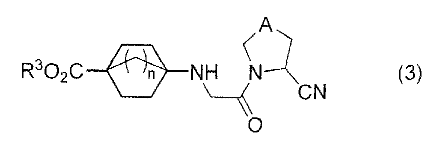 (wherein A represents CH₂, CHF or CF₂;
R³ represents a C₁ to C₆ alkyl group which may have a substituent, a C₃ to C₈ cycloalkyl group which may have a substituent, an arylmethyl group which may have a substituent, an arylethyl group which may have a substituent, an aromatic hydrocarbon group which may have a substituent, an aromatic heterocyclic group which may have a substituent or an aliphatic heterocyclic group which may have a substituent; and
n represents 1 or 2).

11. A method for stabilizing an aminoacetylpyrrolidine carbonitrile derivative represented by the following general formula (1): 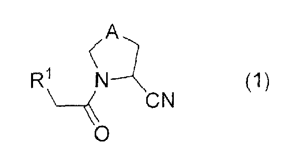 (in the formula (1), A represents CH₂, CHF or CF₂; and
R¹ represents a secondary amino group which may have a substituent), wherein it comprises the step of incorporating, into the aminoacetylpyrrohdine carbonitrile derivative, the following components:
(1) a sugar alcohol;
(2) one or not less than 2 members selected from the group consisting of calcium carbonate, sodium citrate and sodium sulfate; and
(3) a neutral low melting point oily and/or fatty substance.
